(19) Europäisches Patentamt
European Patent Office
Office européen des brevets

(11) Publication number: **0 485 749 A2**

# (12) EUROPEAN PATENT APPLICATION

(21) Application number: **91117535.4**

(22) Date of filing: **15.10.91**

(51) Int. Cl.[5]: **A61K 47/48**

(30) Priority: **15.11.90 US 613360**

(43) Date of publication of application:
**20.05.92 Bulletin 92/21**

(84) Designated Contracting States:
**CH DE FR GB IT LI SE**

(71) Applicant: **BRUNSWICK CORPORATION**
**One Brunswick Plaza**
**Skokie Illinois 60077(US)**

(72) Inventor: **Wrasidlo, Wolfgang A.**
**307 Prospect Street**
**La Jolla, Calif. 92037(US)**

(74) Representative: **UEXKÜLL & STOLBERG**
**Patentanwälte**
**Beselerstrasse 4**
**W-2000 Hamburg 52(DE)**

## (54) Chemical modification of antibodies for creating of immunoconjugates.

(57) A composition of matter is provided having the structure I-L where I is an immunoreceptor and L is a bifunctional linker which is attached to a class of chemical moiety on the immunoreceptor through one functional group and has a free second functional group which is a different class of chemical moiety than the class on the immunoreceptor. The I-L composition has monofunctional chemical reactive groups and is characterized by substantially the same specificity and immunoreactivity as the uncomplexed immunoreceptor. Immunoconjugates having the structure I-L-X are also provided where X is a cytotoxic agent. Methods for producing such compositions are also provided. A method for remitting tumor growth is provided where a therapeutic amount of an I-L-X immunoconjugate is administered to a mammal which selectively binds tumor cell antigens through the immunoreceptor.

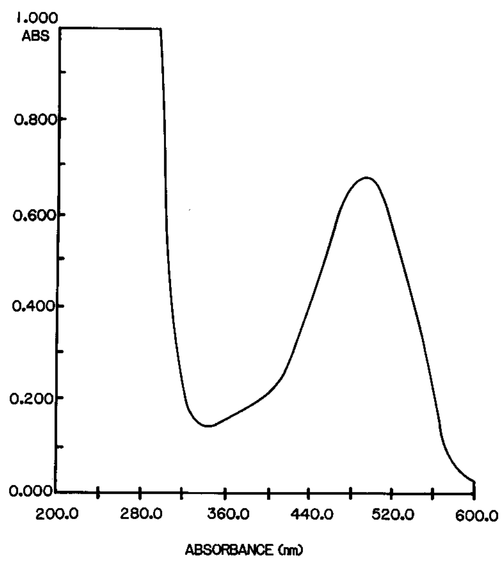


FIG. 2



Rank Xerox (UK) Business Services
(-/2.18/2.0)

## BACKGROUND OF THE INVENTION

In the field of medicine, specifically targeted therapeutic agents have long been perceived as the ideal. The rationale is that freely circulating therapeutic agents will nearly always have some unwanted side effects when administered at concentrations adequate for destruction of the target organism or cell type. The seriousness of side effects becomes quite acute in treating diseases such as cancer where the target is an aberrant population of cells, nearly identical to normal cells of the patient. Specifically targeted therapies would be administered at very low doses and be delivered directly to the target cells, thus minimizing the exposure of normal cells while providing a lethal concentration at the desired site of action.

Currently, one of the most prominent and promising methods for targeting therapeutic agents is the use of monoclonal antibodies. The technology is now well established for the selection of hybridomas which produce monoclonal antibodies that bind specifically to virtually any surface molecular structure on a cell or organism. Mass production facilities can also now produce any desired monoclonal antibody in therapeutic quantities. Such specific targeting agents can be produced which are directed against any bacteria, virus, aberrant cell type or other organism. For example, monoclonal antibodies which specifically recognize aberrant cells such as cancer cells, are possible to produce because these cells express some unique cell surface molecules.

Certain monoclonal antibodies have been shown to have some therapeutic activity by themselves due to effects of their binding to sites which block essential functions. However, this is a relatively rare circumstance and not generally applicable. The complete therapeutic agent must also consist of a cytotoxic agent, such as a drug or radioisotope, and the targeting and cytotoxic agents must be linked together in such a manner that they effectively accomplish the purpose intended. A vast array of cytotoxic agents have already been developed and are available for use. However, an important problem facing development of specifically targeting cytotoxic agents has been linking the targeting and cytotoxic agents together appropriately.

The standard methods of binding an antibody directly to a drug, reacting an antibody, drug and linker molecule simultaneously, or first attaching a linker molecule to a drug and subsequently reacting this activated complex to an antibody have all proven inadequate. The most prevalent problems with these standard methods are the formation of side reaction products and multimeric and cross-linked products. These problems reduce yields, create purification problems and can result in insoluble and inactive complexes.

It is easy to describe what the ideal conjugation of the targeting and cytotoxic agents should be like. The conjugate should be stable during delivery and then be disrupted at the site of action to release active cytotoxic agent. In addition, for production purposes the process of conjugation should proceed rapidly and to near completion with minimal side reactions. The conjugate should be easily purified and be stable for storage until use. These requirements have proven to be far from trivial aspects of the development of such targeted therapeutic agents. Indeed, appropriate monoclonal antibodies and drugs are presently available for many desired applications, but available conjugation strategies are still wanting.

The present disclosure describes a new approach to the conjugation of antibodies to toxic agents which employs the modification of the antibody molecule to fit the appropriate linker chemistries to be employed.

## SUMMARY OF THE INVENTION

A composition of matter is provided having the structure I-L where I is an immunoreceptor and L is a bifunctional linker which is attached to a class of chemical moiety on the immunoreceptor through one functional group and has a free second functional group which is a different class of chemical moiety than the class on the immunoreceptor. The I-L composition has monofunctional chemical reactive groups and is characterized by substantially the same specificity and immunoreactivity as the uncomplexed immunoreceptor. Immunoconjugates having the structure I-L-X are also provided where X is a cytotoxic agent. Methods for producing such compositions are also provided. A method for remitting tumor growth is provided where a therapeutic amount of an I-L-X immunoconjugate is administered to a mammal which selectively binds tumor cell antigens through the immunoreceptor.

## BRIEF DESCRIPTION OF THE DRAWINGS

Figure 1 shows the change in isoelectric point of a monoclonal antibody before (lanes 1 and 2) and after (lanes 3 and 4) attachment to the linker diglycolic anhydride.

Figure 2 demonstrates the conjugation of DXR to the monofunctional chemically reactive monoclonal antibody-linker complex.

Figure 3 shows the reaction kinetics of DXR to a monoclonal antibody diglycolic anhydride linker complex.

Figure 4 shows the reaction kinetics of DXR to a monoclonal antibody-citraconic anhydride linker complex.

Figure 5 shows the immunoreactivity of the unconjugated antibody (open circles), the conjugated antibody (filled circles) and an unconjugated non-specific antibody (squares).

Figure 6 shows the immunoreactivity determined by flow cytometry of unconjugated (open circles) and conjugated (filled circles) antibodies on A172 target cells and the unconjugated (triangles) and conjugated (squares) antibodies on T986 non-target cells.

Figure 7 shows the cytoxicity of DXR alone on DAOY (x) and U251 (t) cell lines and of the conjugated antibodies on DAOY (circles) and U251 (squares) cell lines.

Figure 8 shows the targeting of tumor cells in nude mice using DXR immunoconjugates; saline control (filled circles); unconjugated LM609 monoclonal antibody (open circles); DXR alone (open squares); DXR and LM609 together (filled squares); DXR conjugated to a non-specific antibody (H36-DXR, triangles) and LM609-DXR immunoconjugate (x).

## DETAILED DESCRIPTION

This invention is directed to a simple and efficient method for producing immunoconjugates. The method employs bifunctional linkers which are first coupled to an immunoreceptor to produce an immunoreceptor-linker compound which has monofunctional chemical reactive groups. The monofunctional reactive groups are advantageous in that they can be reacted with a cytotoxic agent without cross linking of immunoreceptors and unwanted by-product formation. This greatly improves yields of the immunoconjugate. The immunoconjugates are stable for use in targeting to any cell type in which there is an immunoreceptor which specifically recognizes an antigen on the desired cell type. Thus, the immunoconjugates produced are applicable in suppressing tumor growth in culture and in mammals.

A preferred embodiment of the present invention entails the modification of the amino-terminal sidechain of lysines in a monoclonal antibody (MAb) by chemical reaction with a bifunctional linker molecule, such that an appropriate moiety on the linker molecule remains available for reaction with the desired cytotoxic agent. The resultant MAb-linker compound is, or can be, chemically modified to be monofunctional and reactive with the desired chemical moiety of the desired drug. The final MAb-linker-drug conjugate is stable during delivery to the site of action (including circulation in the blood stream) and is hydrolytically or enzymatically cleavable when internalized by the target cell or organism, releasing active drug or drug derivative. This preferred embodiment can be generalized by the following formula: I-L-X, where I is an immunoreceptor, L is a bifunctional linker which renders the I-L complex chemically monofunctional for attachment of X, a cytotoxin.

As used herein, the term "immunoreceptor" refers to a protein of the immune system which exhibits selective binding to a target cell. The immune protein may be, for example, monoclonal antibodies, polyclonal antibodies and T-cell receptors. The term is also meant to include functional fragments of immunoreceptors. "Functional fragments", as used herein, refers to fragments which exhibit the selective binding properties as that of the intact molecule to a target cell. Selective binding includes binding specificity and affinity of the immunoreceptor for the target cell. The binding event can be, for example, to a cell surface molecule such as a protein antigen. Binding to other cell surface molecules can include antigens such as carbohydrate, lipid, inorganic molecules, polypeptide and peptide so long as the immunoreceptor exhibits selective binding for these antigens. The target cell can be any cell or population of cells which contains a cell surface molecule capable of being bound by the immunoreceptor. This includes cells in culture as well as cells within an organism.

As used herein, the term "linker" refers to a molecule which forms a bridge-like structure between an immunoreceptor and a cytotoxic agent. The linker is used to modify the immunoreceptor so that the immunoreceptor-linker complex has chemical reactive groups which are monofunctional. The term "bifunctional linker" as used herein refers to linkers which contain at least two functional groups for the purpose of coupling a cytotoxic agent to an immunoreceptor. One functional group is used, for example, to form a bond between an immunoreceptor and the linker. The second functional group is used to form a bond between the immunoreceptor-linker complex and a cytotoxic agent. Such linkers can be, for example, diglycolic anhydride and citraconic anhydride.

As used herein, the term "functional group" refers to a chemical moiety which can be used for covalent bond formation and which exhibits a specified chemical reactivity. The specified chemical reactivity defines what type of functional group the chemical moiety belongs to and what type of chemistry can be used in bond formation. For example, carboxylic acids are reactive with primary amines but are not reactive with themselves. Chemical reactivity, such as exhibiting bond formation activity with primary amines, defines the type of functional group carboxylic acids constitute. Similarly, primary amines and hydroxyl groups are reactive with anhydrides such as diglycolic and citraconic anhydride. This specified chemical reactivity places primary amines and hydroxyl groups in the same functional group since both are reactive with anhydrides. Functional groups are found on immunoreceptors, bifunctional linkers and cytotoxic agents.

As used herein, the term "monofunctional chemical reactive groups" refers to two or more chemical moieties which have similar chemical reactivity and therefore are in the same functional group. An immunoreceptor with monofunctional chemical reactive groups has two or more chemical moieties which exhibit monofunctionality. For example, carboxylic acid moieties of glutamic and aspartic acid residues exhibit monofunctionality when considered by themselves. However, when considered in conjunction with primary amine moieties of lysine residues on an immunoreceptor, the two types of moieties, carboxylic acids and primary amines, do not exhibit monofunctionality. Primary amine moieties of lysine residues will react with carboxylic acid moieties but carboxylic acid moieties will not react with themselves.

The invention provides an immunoreceptor, I, attached to a bifunctional linker, L, which has the structure I-L. Also provided are methods of producing such a compound. The bifunctional linker is attached to a class of chemical moiety on the immunoreceptor through a functional group on the linker. The attached linker has a second functional group which is a different class of chemical moiety than the class on the immunoreceptor to which the linker is attached to produce an immunoreceptor with monofunctional chemical reactive groups. The monofunctional chemical reactive immunoreceptor is characterized by substantially the same specificity and immunoreactivity as the uncomplexed immunoreceptor.

The immunoreceptor to which the bifunctional linker is attached is preferably a monoclonal antibody but can be polyclonal antibodies or other immune system proteins which exhibit selective binding to a target cell. Monoclonal antibodies are beneficial since they can be generated to selectively bind almost any desired antigen and can be produced in large quantities.

Creation of monofunctional groups is desired when at least one of the chemical moieties on a cytotoxic agent or immunoreceptor being used to form a bond is also found on either of the two reactants. This situation leads to bond formation between immunoreceptors and/or cytotoxic agents in addition to the desired immunoreceptor-cytotoxic agent conjugates. An example of this is the attachment of a cytotoxic agent through a primary amine to a carboxyl group on an immunoreceptor. In addition to the desired amide bond formed between the cytotoxic agent and immunoreceptors, amide bonds will also be formed between primary amines located on one immunoreceptor with carboxyl groups located on a second immunoreceptor.

To alleviate cross linking reactions such as that described above, the present invention provides for linkers which can react with chemical moieties of one functional group and thereby confer a different chemical reactivity of a second functional group. Changing the chemical reactivity of a functional group to a second type of functional group results in monofunctionality between the two chemical moieties. This additionally provides for a greater number of sites in which to attach a cytotoxic agent.

A specific example of generating monofunctionality between two functional groups is the attachment of a citraconic anhydride linker to a primary amine found on an immunoreceptor through the following reaction scheme:

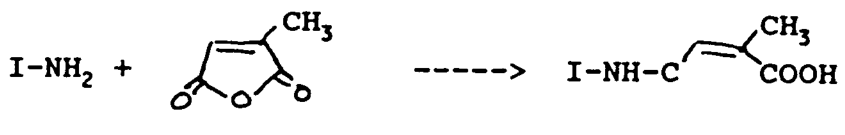

where $I\text{-}NH_2$ designates a primary amine of an immunoreceptor.

The above reaction yields an immunoreceptor-linker compound in which the primary amines have been converted to carboxylic acid moieties. The newly converted primary amines are now monofunctional with carboxyl moieties on the immunoreceptor and available for use in bond formation with, for example, the primary amines of many cytotoxic agents without cross linking reactions.

Bifunctional linkers other than citraconic anhydride can be used which accommodate the need for various moieties present on different cytotoxic agents as well as differences in chemical conditions necessitated by additional functional groups present on cytotoxic agents. For example, other anhydrides

displaying similar chemical reactivity as that of citraconic anhydride can also be used and include, for example, diglycolic anhydride, dimethylmaleic anhydride and cisaconitic anhydride. The reaction of any of these linkers with primary amines on an immunoreceptor is similar to that of citraconic anhydride and is shown below for diglycolic anhydride.

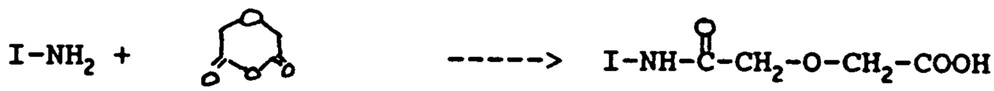

Additionally, bifunctional linkers, including isocyanides and chloroformates, can be used to convert chemical moieties other than primary amines to monofunctional reactivity with respect to a second type of chemical moiety. These additional linkers therefore expand the types of potential chemical moieties on the immunoreceptor which can be used to create a molecule with monofunctional chemical reactivity. The chemical moieties which can be used include aldehyde groups on carbohydrate chains, phenolic hydroxyl groups of tyrosines and sulfhydryl groups of cystines. The types of linker to be used will depend on the functional groups contained on the cytotoxic agent and immunoreceptor which will be used for bond formation and also on additional functional groups contained on either molecule which can cross-react. The chemistries and linkers can be selected by one skilled in the art to create the desired immunoreceptor with monofunctional chemical reactive groups.

The invention provides an immunoreceptor conjugated through a bifunctional linker to a cytotoxic agent and provides for methods of producing such immunoconjugates. The bifunctional linker is attached to a class of chemical moiety on the immunoreceptor through one functional group and has a second functional group which is a different class of chemical moiety than that on the immunoreceptor to produce an immunoreceptor with monofunctional chemical reactive groups. The monofunctional chemical reactive groups are attached to a cytotoxin to produce an immunoconjugate of the structure I-L-X, where I is the immunoreceptor, L is the bifunctional linker and X is the cytotoxin. The immunoconjugate is characterized as having substantially the same specificity as the unconjugated immunoreceptor.

The immunoreceptor-linker compounds containing monofunctional chemical reactive groups described above can be reacted with a variety of cytotoxic agents to produce immunoconjugates specific for a desired target cell and useful for destroying such target cells. Doxorubricin (DXR) and danorubicin are examples of very potent cytotoxic agents. Other cytotoxic agents can also be used in the invention and are known to one skilled in the art. Attachment of DXR and other cytotoxic agents containing primary amines as functional groups to immunoreceptor-linker compounds can be accomplished by the following scheme:

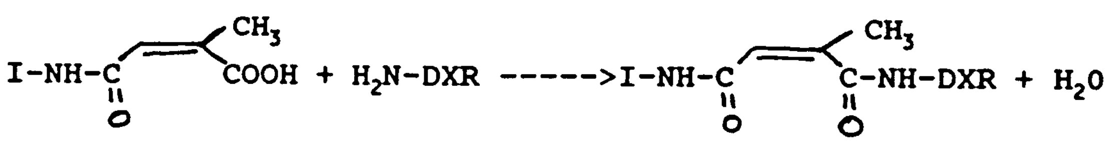

I

II

where compound I represents an immunoreceptor-citraconic anhydride linker complex, $H_2N$-DXR designates a primary amine moiety on doxorubricin and compound II represents the corresponding I-L-X immunoconjugate.

Although there are various chemical pathways to the ultimate goal, the unifying concept remains the initial modification of MAb to create a monofunctional reactive molecule by the addition of a linker molecule which can then be modified, activated or used directly for reaction with the desired chemical moiety on a drug molecule. In the most preferred embodiment of this conjugation strategy, the modification of lysine amino groups is performed on MAb via reaction with an anhydride linker to create MAb-L complexes with reactive carboxylic acid groups. Such a complex is monofunctional with respect to the reaction to link a drug, either directly or following activation, since the carboxylic acid group is much more preferentially reactive than the amino group. The result is that the reaction with the drug proceeds more completely with fewer side reactions and cross linkings.

A priori there is no way to discern if the above preferred strategy or the reverse, i.e., attaching a linker to a drug and then reacting with the MAb, is the best. However, experimentation leading to this disclosure has demonstrated the significant advantages of the strategy described herein in detail. The reverse strategy

results in a plethora of side reactions yielding esters and mixtures of conjugates joined at all possible functional groups. The resultant product is low in specific activity due to high levels of precipitated product (generally cross linked multimers of MAb) and nonactive combinations. The preferred strategy described in this disclosure results in a higher specific activity product at a higher overall yield, and is thus a distinct advantage over previous procedures for creating MAb-drug conjugates.

The invention also provides for bifunctional linkers which are acid labile. All of the above linkers, when used to attach a cytotoxic agent to an immunoreceptor, are stable under physiological conditions of the blood and extracellular matrix, but are unstable at the low pH of about 4 inside the lysosome. Shown below is the mechanism for releasing DXR from an immunoreceptor-citraconic anhydride linker immunoconjugate under acidic conditions.

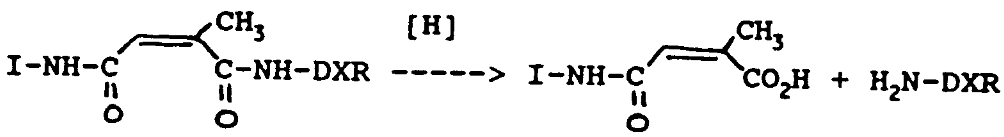


When immunoreceptors bind to antigens on a target cell surface, the immunoreceptor along with whatever is attached to it can be internalized by the cell through endocytosis. Once endocytosed, immunoconjugates such as those of the present invention are directed to an intercellular lysosomal compartment. The pH of this compartment is acidic and aids in the breakdown of macromolecules. Conjugation of a cytotoxic agent through an acid labile linker is advantageous since the agent will be released once inside the compartment and free to perform its intended function. Likewise, linkers which are enzymatically cleavable, for example, can also be used. Such linkers include peptide linkers which can be cleaved by proteases. This mechanism of intercellular delivery is faster and more efficient than using a linker molecule which is not labile.

The I-L compounds and I-L-X immunoconjugates of the present invention are all reacted under conditions which maintain immunoreactivity and binding specificity. The high level of activity and specificity retained compared to the unconjugated immunoreceptor is due to first producing monofunctional chemical reactive groups on the immunoreceptor.

Additionally, one skilled in the art can ensure immunoreactivity and specificity by attaching the linkers and drugs under conditions which protect the binding pocket of the immunoreceptor. For example, in addition to the cross linking and precipitation of immunoreceptors described above which causes loss of activity, chemical moieties within the binding pocket of an immunoreceptor can form a bond with a linker or cytotoxic agent. Forming monofunctional compounds or immunoconjugates through moieties inside the binding pocket can cause a loss in immunoreactivity because such moieties may be necessary for recognition and binding of antigen. This problem can be overcome by attaching linkers and cytotoxic agents to the immunoreceptor which is first bound by antigen. With the binding pocket protected by antigen, any chemical moiety susceptible to bond formation with the linker is physically protected from bond formation. The chemical reactions can be performed, for example, with antigen-bound immunoreceptor in solution, with the immunoreceptor bound to the antigen on an insoluble support or using interfacial condensation techniques, U. S. Patent Application Serial No. 07/419,337, which is incorporated herein by reference. Such methods are known to one skilled in the art.

The invention provides a method of delivering cytotoxic agents to target cells as well as a method of remitting tumor growth consisting of administering to the target cells or mammal a therapeutic amount of an immunoconjugate with the structure I-L-X, where I is an immunoreceptor which selectively binds target cell or tumor cell antigens, L is a bifunctional linker which is attached to a class of chemical moiety on the immunoreceptor through one functional group and has a second functional group which is a different class of chemical moiety than that on the immunoreceptor to produce an immunoreceptor with monofunctional chemical reactive groups. The monofunctional groups are attached to a cytotoxic agent to produce an immunoconjugate characterized as having substantially the same specificity as the unconjugated immunoreceptor.

What is referred to as a cytotoxic agent is a substance which directly exerts cytotoxic action on cells, or can be converted in vivo to a substance which exerts cytotoxic action on cells. Examples of such cytotoxic agents are doxorubicin, daunorubicin and actinomycin D. Such agents can be coupled to any immunoreceptor which is specific for a cell surface antigen. Target antigens may include, for example, viral or bacterial antigens. Derivatives of cytotoxic agents can be prepared as a general agent for coupling to any antibody or

antibody fragment of the desired target specificity. Derivatives can also be prepared for coupling to other types of target cell binding proteins, such as T cell receptors. All of these types of proteins demonstrate binding specificity toward specific target cell antigens.

The immunoconjugates of the present invention can be used to produce cytotoxic effects on target cells. For example, it is possible to use the immunoconjugates to destroy a certain population of cells such as T cells or B cells in an organism by coupling a cytotoxic agent to an immunoreceptor which exhibits binding specificity to the desired population of T or B cells. In a similar manner, a specific cell type may be purified from a contaminating population of cells in culture by treating the culture with an immunoconjugate in which the immunoreceptor has binding specificity for the contaminant cell type. Therefore, the invention provides a method of producing cytotoxic effects on target cells by administering to the target cells an effective amount of an immunoconjugate which exhibits selective binding to target cells. The immunoconjugate may be combined with a pharmaceutically acceptable carrier.

The following examples are intended to illustrate, but not limit, the invention.

## EXAMPLE I

### Conjugation of Monoclonal Ab KS-1/4 to Doxorubricin through a Diglycolic Anhydride Linker

Conjugation of monoclonal antibody (MAb) KS-1/4 to doxorubricin (DXR) was accomplished in a two step process. First, KS-1/4 was coupled, through lysine residues, to the bifunctional linker diglycolic anhydride (DGA) to produce an antibody-linker complex (MAb-L). A 6 ml solution of KS-1/4 (16.9 mg/ml) was adjusted to pH 9.3 and 18 mg of solid diglycolic anhydride was added while stirring. The pH was readjusted to 9.0 until all crystals disappeared. The solution was left stirring for 30 additional minutes by which time the pH had decreased to 8.1. Reactions were monitored by isoelectric focusing of beginning and end products using a Pharmacia Phast System (Pharmacia, Pleasant Hill, CA) as recommended by the manufacturer (Figure 1). A quantitative change in the isoelectric point of KS-1/4 (lanes 1 and 2) from an average pI of 6.8 before coupling to a pI of 5.4 after coupling (lane 3 and 4) was observed.

In a separate series of reactions, alternative conditions were tested for coupling DXR to the above MAb-L complex.

(a) The pH of the MAb-L solution was decreased to 6.1 and 2 mg of solid DXR was added followed by 2 mg of 1-(3-dimethylaminopropyl)-3-ethylcarbodimide hydrochloride (ECDI). The mixture was left stirring for an additional 30 minutes. Purification of the final product (MAb-L-DXR) was achieved using gel filtration on Sephadex G-25. (NAP-5 columns, supplied and used as recommended by the manufacturer; Pharmacia, Pleasant Hill, CA, Catalogue #17-0853-01). The purified conjugate yielded a drug to antibody molar ratio of 3.9 as described in Example III.

(b) A separate aliquot of the conjugated MAb-L solution (1 ml of 10 mg/ml) was adjusted to pH 5.8 prior to addition of 2 mg of ECDI. The clear, colorless solution was stirred for 45 minutes, until the pH remained stable at 6.45. Adjustment of the pH to 6.0 preceded addition of 2 mg DXR-HCl in N-methyl pyrollidone (which was added in three 20 µl portions). The clear, brick red solution was stirred for 1 hour until a pH of 6.6 was reached. Product (0.5 ml diluted with 1.0 ml phosphate buffered saline (PBS)) was purified on Sephadex G-25 as described above. Figure 2 documents the conjugation of DXR to the MAb-L complex. Shown is an ultraviolet spectrum of the final product between 200 and 600 nm wavelength. The two peaks observed within the purified product correspond to maximal absorbances for the individual components (i.e., max. absorbance = 280 nm for the antibody and max. absorbance = 485 nm for DXR).

(c) The pH of the MAb-L solution (1.1 ml of 10 mg/ml) was adjusted to 5.8 prior to addition of 10 mg of said ECDI. The reaction proceeded for 1.5 hours followed by the addition of 2.5 mg of DXR 80 µl neutralized with 3 µl triethylamine (TEA). After 30 minutes, 100 µl of the sample was purified by gel filtration on Sephadex G-50 (NICK columns, supplied and used as recommended by the manufacturer, Pharmacia, Pleasant Hill, CA, Catalogue #(7-0855091). The spectrum between 200 and 600 nm of the purified conjugate was analyzed as described in part (b) above. Peaks were observed at 280 and 485 nm demonstrating the chemical coupling of DXR to the MAb-L complex.

Of the three alternative conditions, the method described in (c) is preferable because it results in the highest drug to antibody molar ratio for coupling.

## EXAMPLE II

### Conjugation of Monoclonal Antibody LM609 with Doxorubricin through Diglycolic Anhydride

The monoclonal antibody LM609 was reacted with the linker molecule diglycolic anhydride (DGA) using analogous reaction conditions as described in Example I. First, the pH of LM609 (1.6 mls of 11.5 mg/ml) was adjusted to 10.5. DGA (24 mg) was added while maintaining the pH at 9.0 with 1 N NaOH. The reaction was incubated for one hour at room temperature and then purified by gel filtration of Sephadex G-25 as described in Example I to yield a solution of MAb-L complexes. The purified product was adjusted to pH 5.5 and reacted with a 300-fold molar excess (7 mg) of ECDI for 90 minutes. A 30-fold molar excess of DXR (2 mg) was added to the MAb-L complexes. Prior to addition, 2 mg of DXR was solubilized in 99 µl of dimethylacetamide containing 1 µl TEA. The reaction proceeded at room temperature and is shown in Figure 3. Upon addition of DXR, the solution immediately increased to pH 6.7 and a 0.5 ml aliquot was removed for purification. The resultant MAb-L-DXR complex was purified by gel exclusion chromatography on G-25 as described in Example I.

## EXAMPLE III

### Coupling of DXR through a Citraconic Anhydride Linker Moiety to LM609

In this example, citraconic anhydride was used as the chemical moiety in which to attach DXR to the monoclonal antibody LM609. Parallel reactions were performed to determine the optimal molar ratios for coupling citraconic anhydride to the MAb. The reactions consisted of addition of a (a) 50- (b) 100- (c) 200- and (d) 500-fold molar excess of citraconic anhydride to LM609. Specifically, addition of (a) 0.38 mg in 5 µl, (b) 0.76 mg in 10 µl, (c) 1.51 mg in 20 µl and (d) 3.79 mg in 50 µl, respectively, of citraconic anhydride (from a dimethyl acetamide stock (DMAc)) was added to 1 ml (10.5 mg) of LM609. The quantities were mixed at a pH of 9.3-9.8 and the pH adjusted to 8.7-9.2 after their addition. Reactions continued for one hour at room temperature while maintaining the pH below 9.0. Products were purified by gel filtration on Sephadex G-50 as described in Example I.

For the activation and subsequent coupling to DXR, each of the above MAb-L complexes were reacted with an excess of ECDI. Briefly, 1.5 mg of ECDI was added to all four solutions and reacted for two hours until the pH reached 8.0. DXR (5 mg) was dissolved in 200 µl N-methylpyrollidone (NMP) containing 2 µl TEA and 50 µl aliquots dispensed into each of the above four MAb-L solutions. The pH decreased to 7.4-7.2. Reactions proceeded overnight at room temperature. A precipitate could be visualized after two hours of incubation and was removed by centrifugation at 1000 rpm for five minutes.

To measure the extent of coupling, absorbances were taken at 485 nm and 280 nm and the ratio calculated. Shown in Table I are the drug-to-antibody coupling ratios ($Abs_{280}/Abs_{485}$) for each of the four conditions. Coupling of DXR to monoclonal LM609 was observed for all citraconic anhydride rations used. The optimal molar ratio was reaction 3 (200 moles linker: 1 mole Antibody).

TABLE I

| Coupling of Citraconic Anhydride to Monoclonal LM609 | | | | |
|---|---|---|---|---|
| Reaction | Moles Antibody | Moles Citraconic | Molar Excess | Drug to Antibody Ratio $A_{280}/A_{485}$ |
| 1 | $6.77 \times 10^{-8}$ | $3.38 \times 10^{-6}$ | 50 | 8.3 |
| 2 | $6.77 \times 10^{-8}$ | $6.77 \times 10^{-6}$ | 100 | 16.0 |
| 3 | $6.77 \times 10^{-8}$ | $1.75 \times 10^{-5}$ | 200 | 25.1 |
| 4 | $6.77 \times 10^{-8}$ | $3.38 \times 10^{-5}$ | 500 | 15.0 |

EXAMPLE IV

Coupling of DXR through a Citraconic Anhydride Moiety to Monoclonal Antibody 9.2.27

A 3 ml solution of monoclonal antibody 9.2.27 (10 mg/ml) was adjusted to pH 9.5 with 1 N NaOH. The pH was maintained at 9.0 while 35 µl of citraconic anhydride stock (136-fold molar excess) in NMP was added. After five minutes, the pH was lowered to 8.5. The clear solution was stirred for an additional one hour and the resultant MAb-L product purified by gel filtration chromatography on G-25 as described in Example I.

For activation and coupling of DXR to the MAb-L complex, 2.9 ml of the above purified MAb-L complex was adjusted to pH 6.0 followed by addition of 3.3 mg of ECDI. The reaction proceeded for 2 hours at room temperature while stirring. A 25-fold molar excess of DXR was added (35 ml of a 25.4 mg/ml in 100 µl DMAc, 3 µl TEA (which consists of 0.5 ml DMAc and 3 ml TEA). The red, clear solution was reacted for 18 hours at room temperature. Chromatography on G-25 was performed as described above to separate the MAb-L-DXR end product from contaminants.

A parallel conjugation was performed to monitor the kinetics of DXR coupling to the MAb-L complexes. In this example, 3.9 mg of ECDI was added to the MAb-L complexes, prepared as described above, and the reaction proceeded at pH 7.5. Shown in Figure 4 is a time course for the coupling reaction. The extent of the reactions were measured at each time point by $A_{280}/A_{485}$ ratios (MAb-L-DXR products 268-67a through 267-67e). This Figure demonstrates that 75% of the reaction is complete within the first four hours.

EXAMPLE V

Immunoreactivity of Conjugated Antibodies Determined by ELISA

To assess whether the 9.2.27 DXR conjugated antibodies (MAb-L-DXR products from Example IV) retained binding activity and antigen specificity, an ELISA was performed on a M21 melanoma cell line (Figure 5). This cell line expresses specific antigens which are recognized by the monoclonal 9.2.27.

Appropriate cell lines that were in logarithmic growth phase were harvested using 1 mM EDTA. The cells were centrifuged at 1000 x g and rinsed two times with 1 x PBS. The cell pellet was resuspended in 1 x PBS in volume to equal 1 x $10^6$ cells/ml. Using a 12 channel micropipettor, cells were platted out in 50 µl/well volume to equal 5 x $10^4$ cells/well in flexible 96 well plates (Falcon #3912, Becton Dickinson & Co.). The 96 well plates were placed in a dry 37° C incubator for 3 to 5 days till wells were completely dry. The new plates were tested with purified monoclonal antibody standards before using in specificity assays.

The above prepared dry cell plates (both target and non-target cells) were coated with 200 µl of 0.5% BSA in PBS (0.5% BSA/PBS). The plates were placed on a shaker for one hour at room temperature. Following incubation for one hour, the plates were washed with 0.5% BSA/PBS in the following manner. For the first wash, 300 µl of 0.5% BSA/PBS was added and immediately flicked out followed by slapping dry on a teri towel. For the remaining wash, 300 µl of 0.5% BSA/PBS was added and allowed to incubate at room temperature for 3-5 minutes before flicking dry. Following the final wash, the plates were slapped dry on a teri towel.

To the above prepared and washed plates, standards and samples were added in 100 µl volumes/well starting at 10 µg/ml with serial dilutions down to 1 pg/ml. The samples were diluted in 0.5% BSA/PBS. The samples were incubated for 1 hour at room temperature while shaking. Following incubation, the plates were washed as described above and 100 µl/well of secondary antibody (goat anti-mouse coupled to horseradish peroxide) was added at 1:4000 dilution in 0.5% BSA/PBS. The antibody was allowed to bind for 30 minutes while shaking at room temperature. The plates were again washed as described above and peroxidase staining was developed for 5-20 minutes with shaking by addition of 100 µl/well of OPD/Phos-citrate buffer (40 ml of phosphate-citrate buffer, 12 mg o-phenylene-diamine, 6 µl $H_2O_2$. The plates were quenched by addition of 50 µl/well of 4N sulfuric acid and read at 490 nm on a titertek Multiskan MCC/340 ELISA reader.

The results demonstrate that both the unconjugated and conjugated antibodies both specifically bind the M21 cell line compared to control antibody (KS 1/4). Moreover, the MAb-L-DXR conjugate retained nearly all its functional activity (compare 9.7.27 to 268.67b) demonstrating that the chemical procedures and moieties described do not have any detrimental effects on antigen binding and specificity.

EXAMPLE VI

Immunoreactivity of Conjugated Antibodies determined by Flow Cytometry

Immunoreactivity of the conjugated antibodies from Example IV was additionally determined on A172 and T98G glioma cell lines by flow cytometry (FACS) (Figure 6). A172, which expresses the antigen recognized by 9.2.27 and T98G, which does not exhibit 9.2.27 immunoreactivity, were prepared for FACS analysis as described below.

Briefly, the cells were harvested at $5x10^5$ - $1x10^6$ cells per sample and placed on ice in conical tubes. The cells were washed twice in PBS/Azide/BSA (PBS + 0.02% $NaN_3$ + 0.1% BSA), centrifuged at 1000 rpm for 10 minutes and the supernatant was aspirated completely. Various concentrations of the monoclonal antibody 9.2.27 and its DXR-conjugated form (268.67d from Example IV) were added to the cell pellets in 100 µl volumes. No antibody was added for negative controls. The antibodies were incubated with the cells on ice for 30-60 minutes followed by two PBS/Azide/BSA washes as described above. After the final centrifugation, fluorescein isothiocyanate (FITC)-conjugated goat anti-mouse IgG/IgM (Boehringer Mannheim, Indianapolis, IN) was added to each sample in 100 µl volumes at a 1:50 dilution. The FITL-conjugated antibody was incubated on ice in the dark for 30-60 minutes followed by two PBS/Azide/BSA washes. The cell pellet was resuspended in 0.5 µl PBS/Azide/BSA and transferred to round bottom tubes. The cells were fixed in 0.5% paraformaldehyde Dead cells were excluded from FACS analysis by prior incubation with > 1 ng/ml propidiumiodide (Sigma, St. Louis, MO) for five minutes and washed once in PBS/Azide/BSA.

The results indicate that both the parental antibody and its conjugated form are specific for the antigen-expressing cell line. The conjugated antibody retained 80% of its immunoreactivity compared to the control.

EXAMPLE VII

Cytotoxicity of DXR-conjugated Antibodies

DAOY and U251 cell lines were cultured in RPM1 tissue culture media supplemented with 10% fetal bovine serum (FBS). Tumor cells ($10^4$) were plated in 100 µl volumes and incubated with the antibody conjugates for two hours. Thereafter, the plates were washed three times in tissue culture media and incubated overnight. Each well received 1µCi of $^3$H-thymidine and after 16 hour incubation, the cells were harvested onto glass fiber filters with a Skatron cell harvester. The filters were placed in Ecolume scintillation solution (ICN, Irvine, CA) and were counted in a scintillation counter. $^3$H-Thymidine incorporation, as percentage of untreated control cells, is used to express cytotoxicity. The results are shown in Figure 7.

EXAMPLE VIII

Cytotoxicity of Conjugated Antibodies in Nude Mice

Nude mice Balb/c (Nu/Nu) (Imdyne Inc., San Diego, CA) were injected with M-21 tumor cells at a density of 2 x $10^6$/ml. The mice were kept for seven days prior to injection of immunoconjugates. Mice were injected with either saline, LM609 (unconjugated control) (500 µg/200 µl), DXR (30 µg/200 µl), DXR (30 µg/200 µl) plus LM609 (500 µg/100 µl) together, H36-DXR (conjugated, non-specific Ab) at 500 µg/100 ml or LM609-DXR (conjugated, specific Antibody) at 500 µg/100 µl. Specifically, in vivo animal tumor studies were carried out in Balb/c (Nu/Nu) athymic mice bearing palpable, measurable s.c. tumors of M-21 cell line. All animals had palpable, measurable tumors of about 3 x 3 mm or 9 $mm^2$ at the time therapy was started. Each individual experiment contained ten animals in each of six arms including a PBS control, antibody alone, drug alone, the immunoconjugate specific for antigen present on M-21, the immunoconjugate not specific for antigen present on M-21, and a combination of antibody mixed with drugs. Each animal received an i.p. injection once a week for two weeks. Animals were observed daily for general well-being and survival. Maximum cross-sectioned diameters were measured every third day using calipers. The product of the two cross-section diameters was determined and recorded as tumor size. The results are graphed in Figure 8. Tumors of those mice which received the control injections grew to sizes in the range of 270 $mm^2$. Those mice which received injections of LM609-DXR had tumors whose growth rate was significantly retarded to a size of 20-[40] $mm^2$ after two weeks of treatment.

Although the invention has been described with reference to the presently-preferred embodiment, it should be understood that various modifications can be made by those skilled in the art without departing from the invention. Accordingly, the invention is limited only by the following claims.

## Claims

1. A composition of matter comprising the structure I-L, wherein I is an immunoreceptor and L is a bifunctional linker attached to a class of chemical moiety on the immunoreceptor through one functional group and having a free second functional group which is a different class of chemical moiety than the class on the immunoreceptor, said composition having monofunctional chemical reactive groups characterized by substantially the same specificity and immunoreactivity as the uncomplexed immunoreceptor.

2. The composition of claim 1, wherein the immunoreceptor is selected from the group consisting of monoclonal or polyclonal antibodies.

3. The composition of claim 1, wherein the bifunctional linker is selected from the group consisting of anhydrides, isocyanides and chloroformates.

4. The composition of claim 1, wherein the class of chemical moiety on the immunoreceptor is selected from the group consisting of primary amine, carboxyl, phenolic hydroxyl and sulfhydryl groups.

5. The composition of claim 1, wherein the different class of chemical moiety than the class on the immunoreceptor is selected from the group consisting of primary amine, carboxyl, phenolic hydroxyl and sulfhydryl groups.

6. A composition of matter comprising the structure I-L-X, wherein I is an immunoreceptor, X is a cytotoxic agent and L is a bifunctional linker attached to a class of chemical moiety on the immunoreceptor through one functional group and having a second functional group which is a different class of chemical moiety than the class on the immunoreceptor, said bifunctional linker attached to said immunoreceptor having monofunctional chemical reactive groups, wherein the monofunctional chemical reactive groups are attached to X, said composition characterized by substantially the same specificity and immunoreactivity as the unconjugated immunoreceptor.

7. The composition of claim 6, wherein the immunoreceptor is selected from the group consisting of monoclonal or polyclonal antibodies.

8. The composition of claim 6, wherein the bifunctional linker is selected from the group consisting of anhydrides, isocyanides and chloroformates.

9. The composition of claim 6, wherein the class of chemical moiety is selected from the group consisting of primary amine, carboxyl, phenolic hydroxyl and sulfhydryl groups.

10. The composition of claim 6, wherein the different class of chemical moiety than the class on the immunoreceptor is selected from the group consisting of primary amine, carboxyl, phenolic hydroxyl and sulfhydryl groups.

11. A method of producing an immunoreceptor linker complex comprising attaching a bifunctional linker to a class of chemical moiety on an immunoreceptor through one functional group, said bifunctional linker having a second functional group which is a different class of chemical moiety than the class on the immunoreceptor to produce an immunoreceptor with monofunctional chemical reactive groups characterized by substantially the same specificity and immunoreactivity as the uncomplexed immunoreceptor.

12. The method of claim 11, wherein the immunoreceptor is selected from the group consisting of monoclonal and polyclonal antibodies.

**13.** The method of claim 11, wherein the bifunctional linker is selected from the group consisting of anhydrides, isocyanides and chloroformates.

**14.** The method of claim 11, wherein the class of chemical moiety on the immunoreceptor is selected from the group consisting of primary amine, carboxyl, phenolic hydroxyl and sulfhydryl groups.

**15.** The method of claim 11, wherein the different class of chemical moiety than the class on the immunoreceptor is selected from the group consisting of primary amine, carboxyl, phenolic hydroxyl and sulfhydryl groups.

**16.** A method of producing an immunoconjugate comprising:

(a) attaching a bifunctional linker to a class of chemical moiety on an immunoreceptor through one functional group, said bifunctional linker having a second functional group which is a different class of chemical moiety than that on the immunoreceptor to produce an immunoreceptor with monofunctional chemical reactive groups; and

(b) attaching to the monofunctional chemical reactive groups a cytotoxic agent to produce an immunoconjugate having the structure I-L-X, wherein I is an immunoreceptor, L is a bifunctional linker and X is a cytotoxic agent, said immunoconjugate being characterized by substantially the same specificity and immunoreactivity as the unconjugated immunoreceptor.

**17.** The method of claim 16, wherein the immunoreceptor is selected from the group consisting of monoclonal or polyclonal antibodies.

**18.** The method of claim 16, wherein the bifunctional linker is selected from the group consisting of anhydrides, isocyanides and chloroformates.

**19.** The method of claim 16, wherein the class of chemical moiety on an immunoreceptor is selected from the group consisting of primary amine, carboxyl, phenolic hydroxyl and sulfhydryl groups.

**20.** The method of claim 16, wherein the different class of chemical moiety than that on the immunoreceptor is selected from the group consisting of primary amine, carboxyl, phenolic hydroxyl and sulfhydryl groups.

**21.** A method of remitting tumor growth comprising administering to a mammal a therapeutic amount of the composition of claim 9 which selectively binds tumor cell antigens through the immunoreceptor.

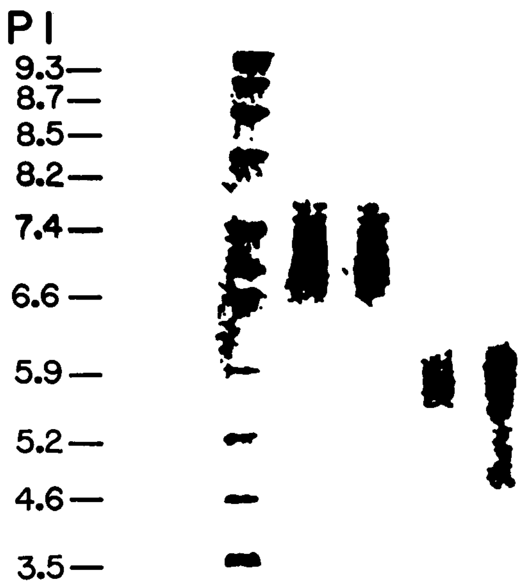


FIG. 1

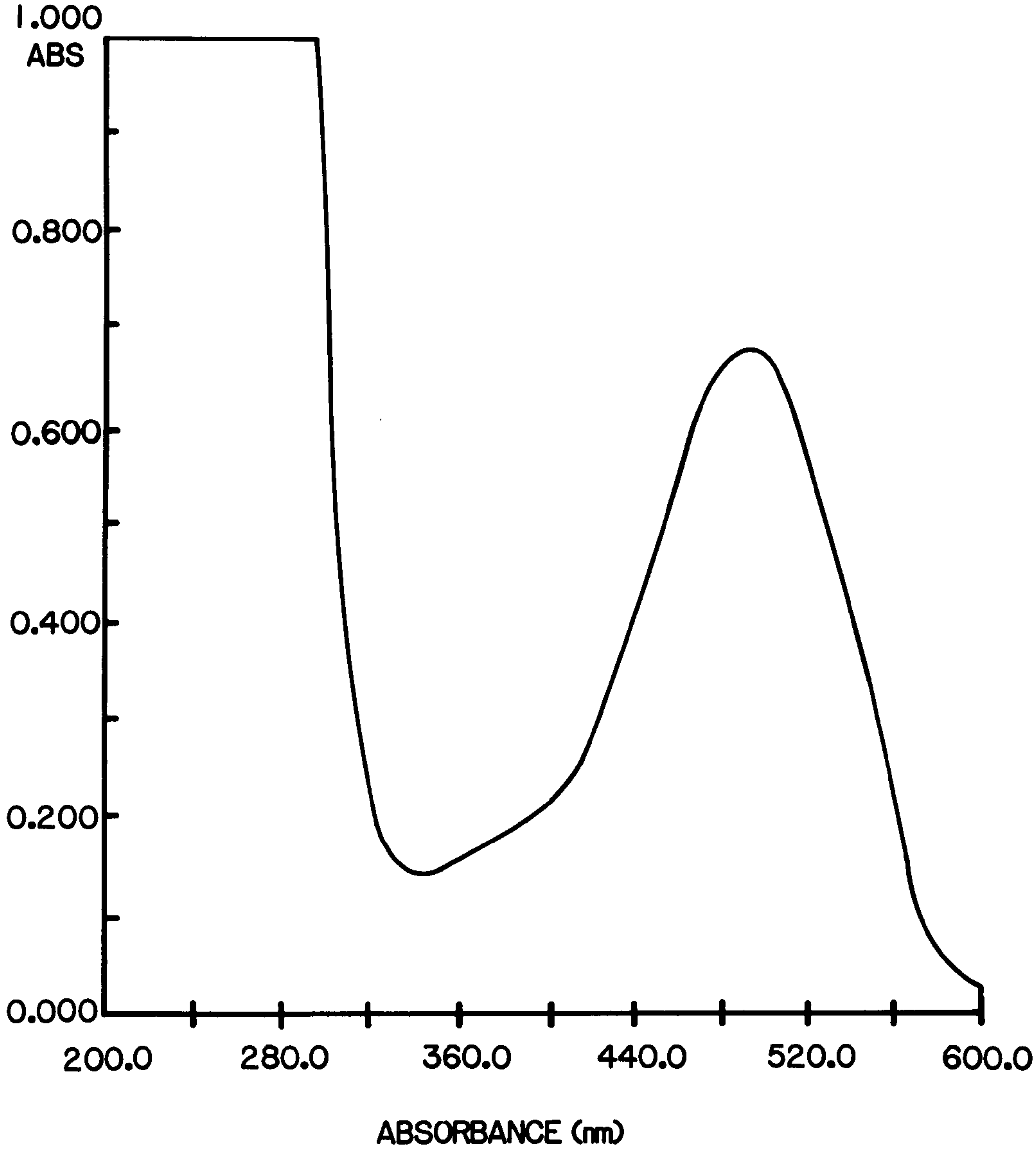
1.000
ABS
0.800
0.600
0.400
0.200
0.000
200.0
280.0
360.0
440.0
520.0
600.0
ABSORBANCE (nm)

FIG. 2

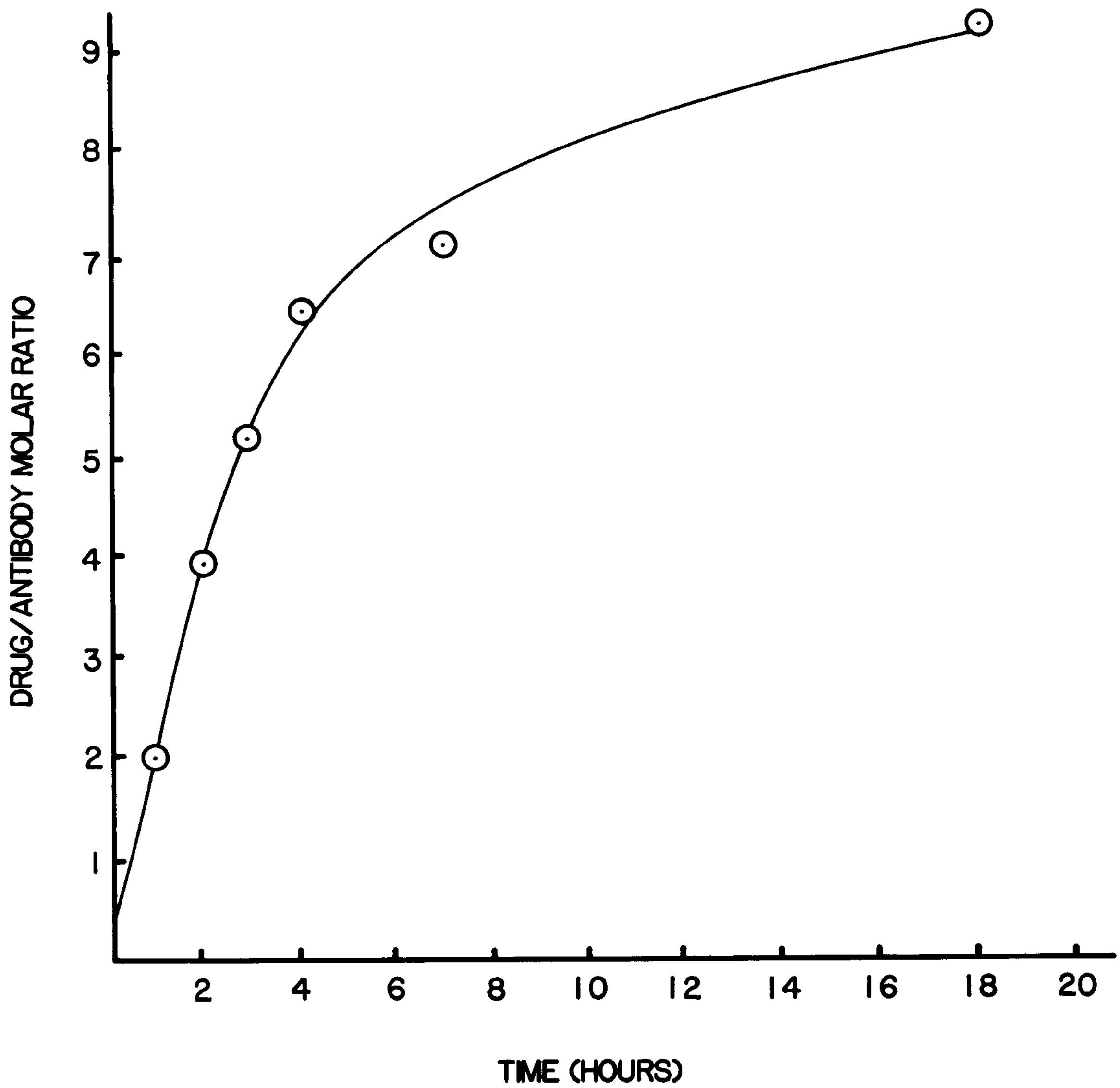
DRUG/ANTIBODY MOLAR RATIO
1
2
3
4
5
6
7
8
9
2
4
6
8
10
12
14
16
18
20
TIME (HOURS)

FIG. 3

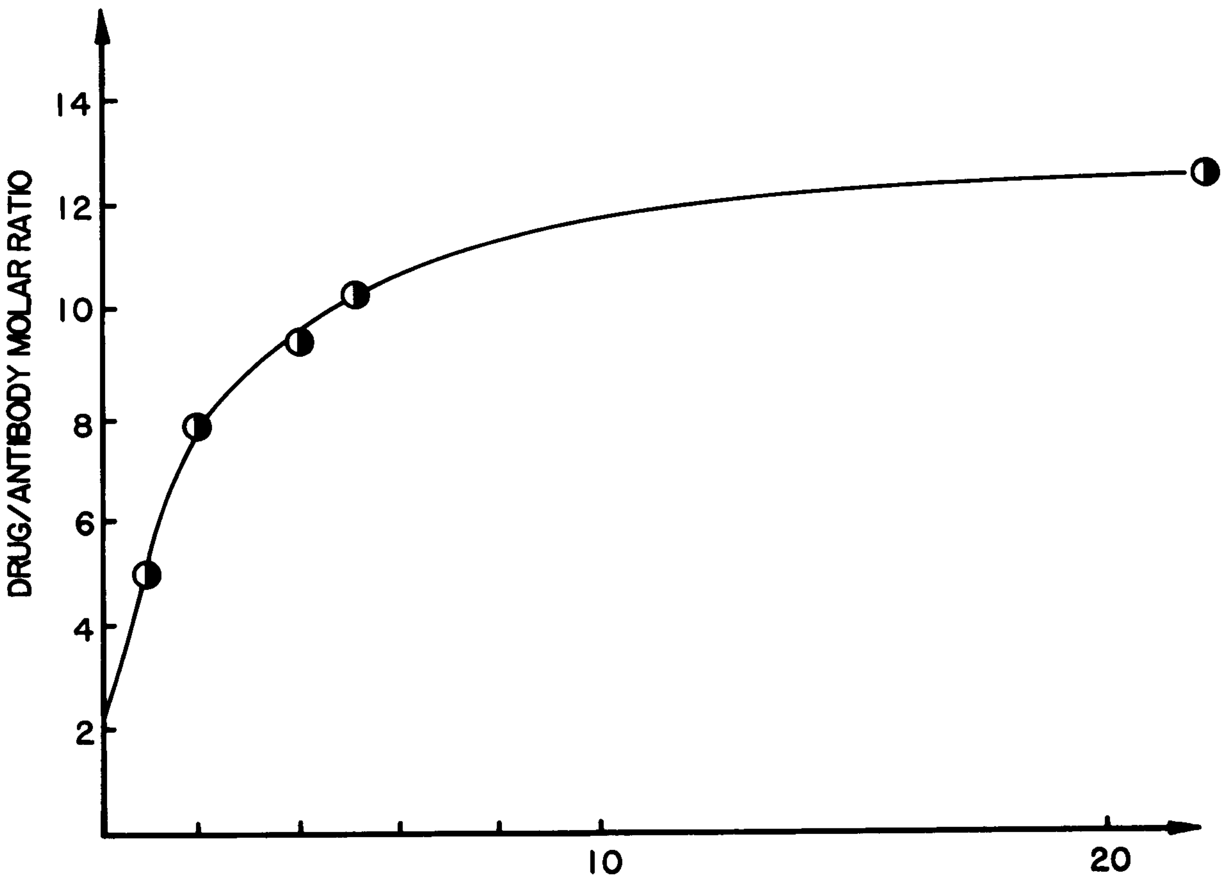


FIG. 4

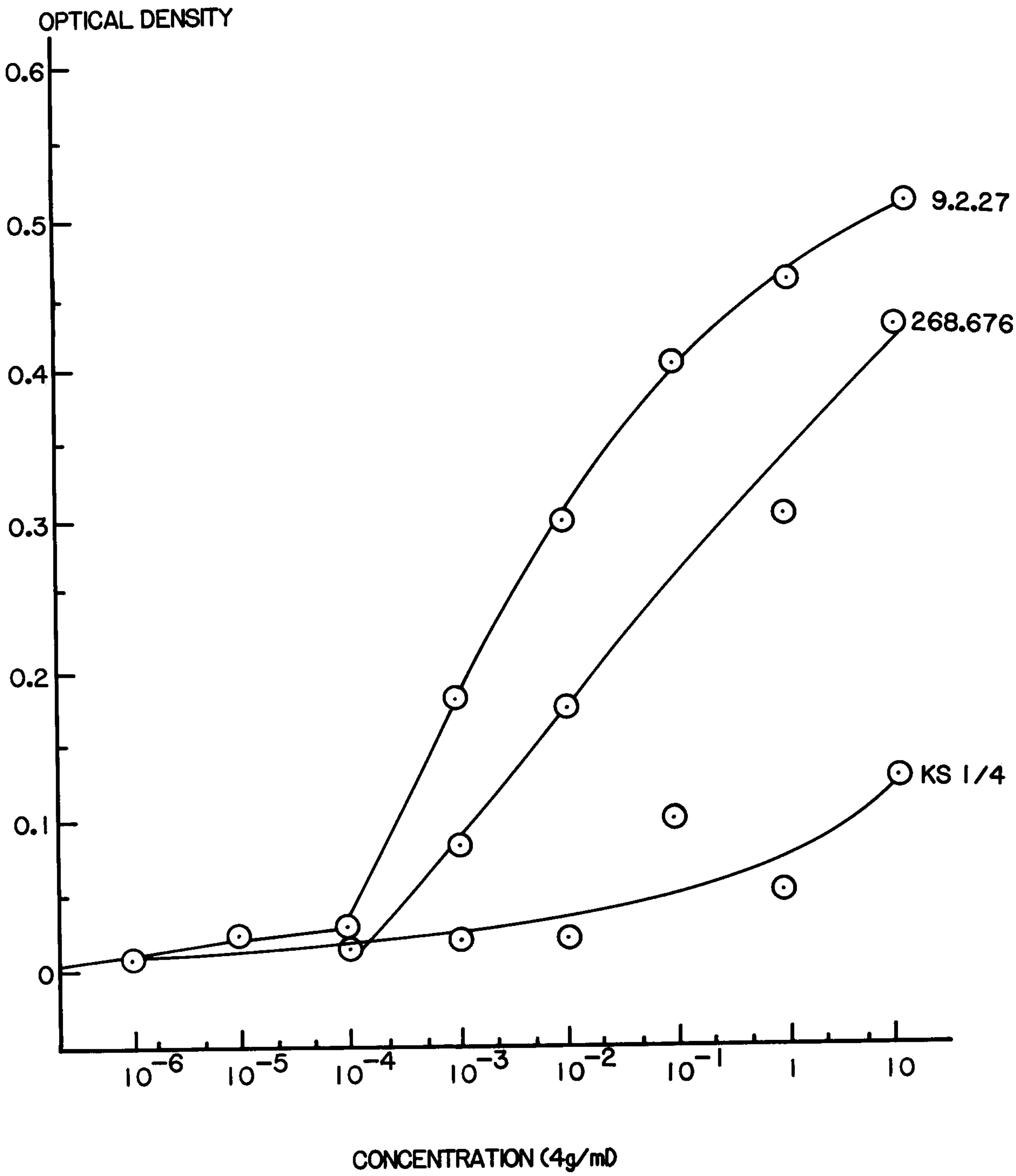
OPTICAL DENSITY
0.6
0.5
0.4
0.3
0.2
0.1
0
9.2.27
268.676
KS 1/4
10^-6
10^-5
10^-4
10^-3
10^-2
10^-1
1
10
CONCENTRATION (4g/ml)

FIG. 5

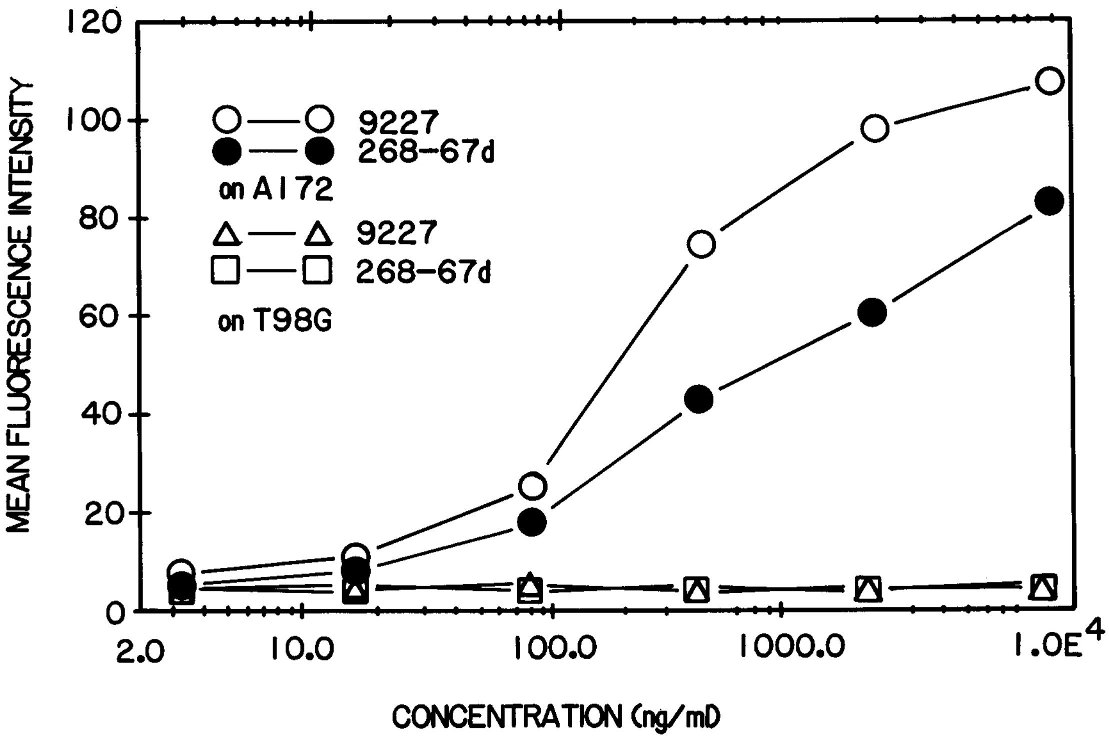
MEAN FLUORESCENCE INTENSITY
120
100
80
60
40
20
0
9227
268-67d
on A172
9227
268-67d
on T98G
2.0
10.0
100.0
1000.0
1.0E4
CONCENTRATION (ng/ml)

FIG. 6

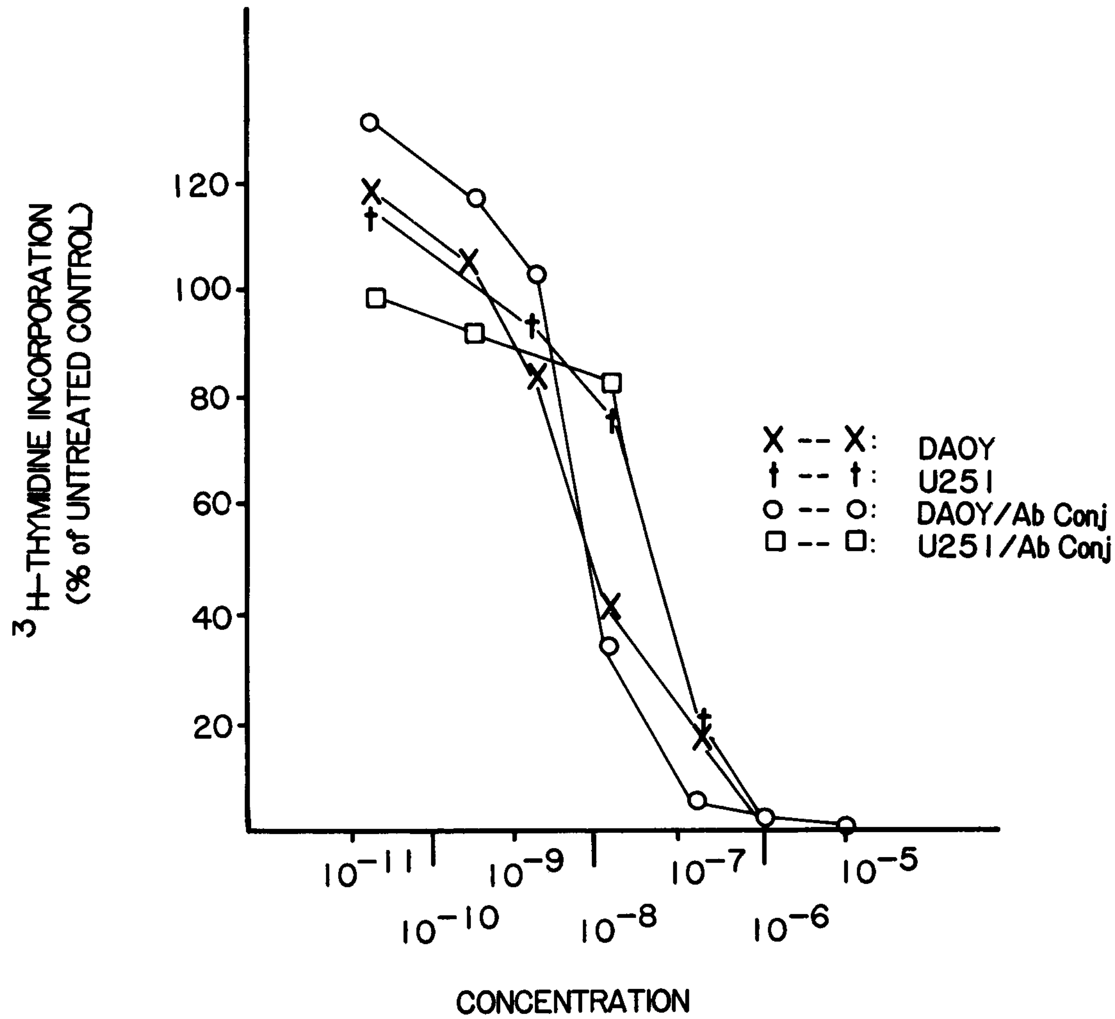
120
100
80
60
40
20
$^3$H-THYMIDINE INCORPORATION
(% of UNTREATED CONTROL)
X -- X: DAOY
† -- †: U251
O -- O: DAOY/Ab Conj
□ -- □: U251/Ab Conj
$10^{-11}$
$10^{-10}$
$10^{-9}$
$10^{-8}$
$10^{-7}$
$10^{-6}$
$10^{-5}$
CONCENTRATION

FIG. 7

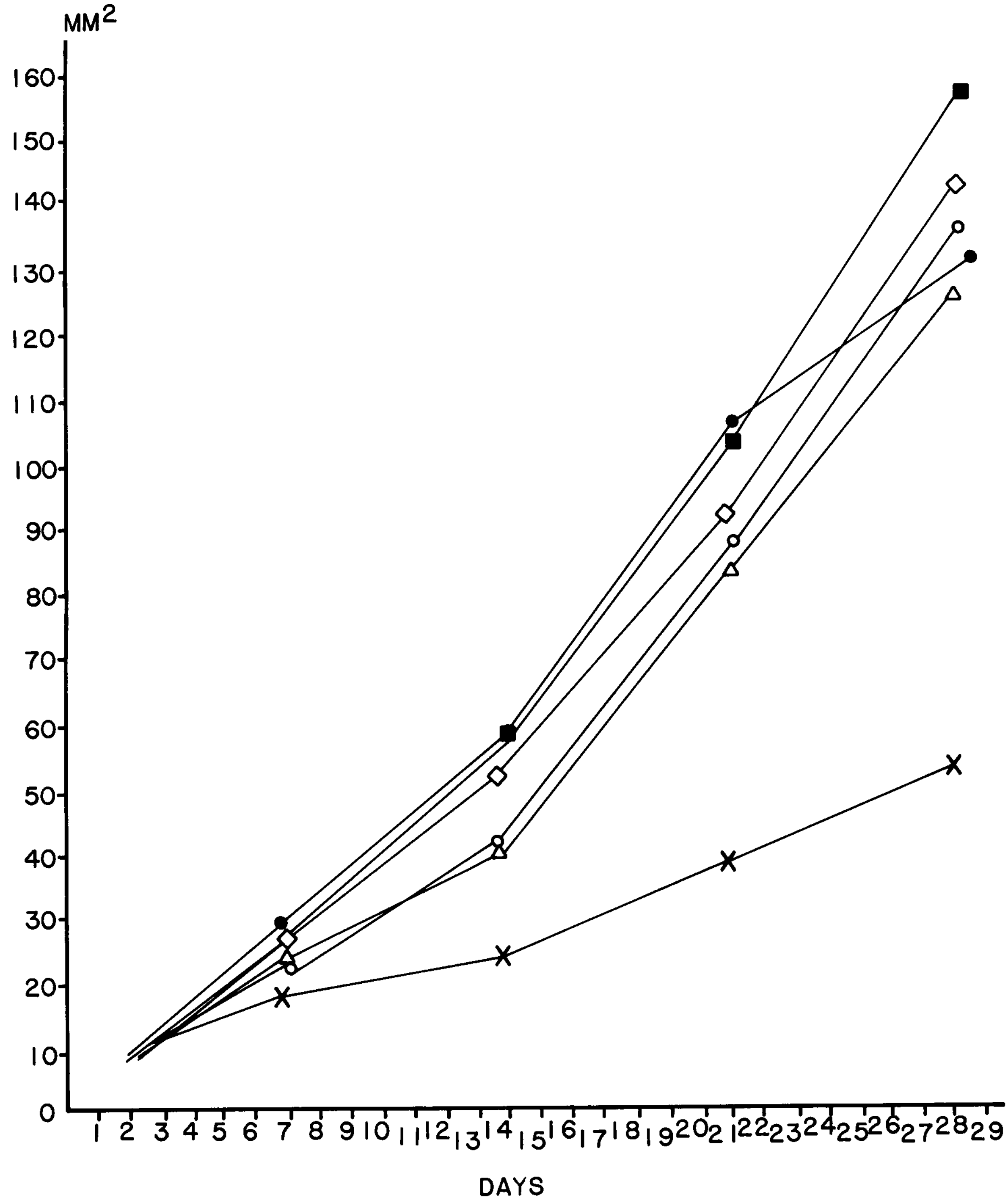
MM2
160
150
140
130
120
110
100
90
80
70
60
50
40
30
20
10
0
1 2 3 4 5 6 7 8 9 10 11 12 13 14 15 16 17 18 19 20 21 22 23 24 25 26 27 28 29
DAYS

FIG. 8